# EUROPÄISCHE PATENTSCHRIFT

(11) **EP 0 941 713 B1**
(45) Veröffentlichungstag und Bekanntmachung des Hinweises auf die Patenterteilung: **03.11.2004**
(21) Anmeldenummer: 98103804.5
(22) Anmeldetag: 04.03.1998
(51) Int. Cl.: A61F 2/06

(54) **Vorrichtung zum Einführen einer Endoprothese in einen Katheterschaft**
Device to insert an endoprosthesis into a catheter shaft
Dispositif pour introduire une endoprothèse dans le tube d' un cathéter

(43) Veröffentlichungstag der Anmeldung: 15.09.1999
(73) Patentinhaber: Schneider (Europe) GmbH, 8180 Bülach (CH)
(72) Erfinder: Gianotti, Marc, 8542 Wiesendangen (CH)
(74) Vertreter: Althoff, Gerhard

(56) Entgegenhaltungen:
- EP-A- 0 119 688
- EP-A- 0 701 800
- EP-A- 0 786 267
- WO-A-96/37167

## Beschreibung

Die Erfindung betrifft eine Vorrichtung zum Einführen einer rohrförmigen, selbstexpandierenden Endoprothese in einen Katheter, mit einem Gehäuse zur Aufnahme der Endoprothese sowie einer Kolbeneinheit, mittels welcher die Endoprothese in axialer Richtung in dem Gehäuse verschiebbar und in das Lumen eines hohlzylindrischen Aussenkatheters des Katheters einführbar ist.

Die Verwendung von selbstexpandierenden Endoprothesen, wie beispielsweise in der US 4,655,771 beschrieben, zur Behandlung von Gefässkrankheiten ist allgemein bekannt. Hierbei wird zum Beispiel mittels eines länglichen und in das zu behandelnde Gefäss einführbaren Katheters eine am distalen Ende im Lumen desselben angeordnete Endoprothese an vorbestimmter Stelle des Gefässes von dem Katheter freigegeben.

Aus der EP 0 607 468 B1 ist zum Implantieren einer selbstexpandierenden Endoprothese (Stützkörper) in ein Gefäss ein länglicher Katheter bekannt, welcher einen flexiblen Aussenkatheter, einen darin angeordneten flexiblen Innenkatheter sowie eine Katheterspitze umfasst, die zur Ausbildung eines Laderaumes für die Endoprothese mit dem distalen Ende des Innenkatheters über ein Zwischenstück verbunden ist. Bei diesem Katheter wird bei teilweise zurückgezogenem Aussenkatheter die Endoprothese manuell verjüngt und in den vorgesehenen Laderaum eingeführt oder eingesetzt, wobei der Aussenkatheter anschliessend nach und nach über die Endoprothese bis zur Katheterspitze geschoben wird.

Weiterhin ist aus der Druckschrift WO 96/37167 eine Vorrichtung zum Einschieben mehrerer Endoprothesen in das distale Ende eines Katheters bekannt, mittels welchem die einzelnen Endoprothesen jeweils mit den in einem Gefäss (Arterie) gebildeten und den Blutfluss vermindernden Verengungen (Stenose) selbstexpandierend in Eingriff bringbar sind. Die Vorrichtung hat ein zum axialen Einschieben mehrerer Endoprothesen ausgebildetes Gehäuse mit austrittsseitig angeformtem Anschlussteil, eine in axialer Richtung in dem Gehäuse verschiebbare Kolbeneinheit sowie den flexibel ausgebildeten Katheter, welcher einen Innenkatheter, einen darin in Längsrichtung verschiebbar angeordneten und mit einem Kopfstück versehenen Führungsdraht sowie einen Aussenkatheter umfasst, wobei der Aussenkatheter am distalen Ende mit einem in das Anschlussteil des Gehäuses einschiebbaren und zur Aufnahme mehrerer in axialer Richtung hintereinanderliegend angeordneter Endoprothesen etwa hohlzylindrisch ausgebildeten Teilstück versehen ist. Die in dem Gehäuse in Reihe hintereinanderliegenden Endoprothesen werden zunächst mittels der Kolbeneinheit in das distale Ende des Katheters geschoben und mittels des Kopfstücks nacheinander mit den Verengungen in Eingriff gebracht. Ein Komprimieren der Endoprothesen ist bei dieser Vorrichtung nicht vorgesehen.

Aus der EP-A 0 701 800 ist eine Vorrichtung zum Komprimieren sowie zum Einziehen einer an beiden Enden mit mehreren in Umfangsrichtung verteilt angeordneten Hakenelementen versehenen Endoprothese in das distale Ende eines Katheters bekannt. Die Vorrichtung umfasst ein Gehäuse mit einer konisch verjüngend ausgebildeten Einführöffnung sowie einer damit in Verbindung stehenden Ausnehmung zur Aufnahme des Katheters, wobei der Katheter mit einem koaxial darin angeordneten und die Endoprothese in axialer Richtung durchdringenden Führungsrohr versehen ist, welches eine distale Spitze sowie ein in axialer Richtung im Abstand dazu angeordnetes Kopfstück mit daran angeordneter Halterung aufweist, mittels welcher die an dem zugewandten proximalen Ende der Endoprothese angeordneten Hakenelemente beim Komprimieren erfasst und gehalten werden. Eine Implantation oder ein vollständiges Freisetzen der Endoprothese im Lumen des Blutgefässes ist mit diesem Katheter nicht möglich.

Weiterhin ist aus der WO-A 96/39077 eine als Katheter ausgebildete Messvorrichtung für eine in den zylindrischen Innenraum eines Hülsenkörpers einziehbare und auf eine Länge abschneidbare Endoprothese bekannt, wobei die Endoprothese mit vorbestimmter Länge von Hand in das Lumen eines Aussenkatheters einzuführen ist. Spezielle Mittel zum Komprimieren und Einziehen der Endoprothese.in den Hülsenkörper sind auch bei dieser Vorrichtung nicht vorgesehen.

Der Erfindung liegt die Aufgabe zugrunde, eine Vorrichtung gemäss der im Oberbegriff des Anspruchs 1 genannten Gattung dahingehend zu verbessern, dass die in ein Blutgefäss zu implantierende und beispielsweise in Bezug auf ihre Abmessungen vorbestimmte Endoprothese unter Beibehaltung einer sicheren und sterilen Handhabung möglichst schnell und ohne zusätzliche Hilfsmittel in das distale Ende eines Katheters einführbar ist.

Die erfindungsgemässe Vorrichtung ist dadurch gekennzeichnet, dass dem Gehäuse ein zum radialen Komprimieren der Endoprothese ausgebildetes Kopfstück zugeordnet ist, welches eine von dem hohlzylindrischen Innenraum des Gehäuses in Richtung einer in Bezug auf den Innenraum kleiner ausgebildeten. Durch trittsöffnung trichterförmige Ausnehmung aufweist.

Hierdurch ist es möglich, die Endoprothese hinsichtlich des äusseren Durchmessers gleichmässig und sukzessive zu komprimieren und in das Lumen des Aussenkatheters einzuführen. Das Montieren der Endoprothese mit der erfindungsgemässen Vorrichtung ist gegenüber der Montage von Hand wesentlich einfacher zu handhaben, wobei eine nachträgliche Sterilisation der Endoprothese hierbei nicht erforderlich ist.

Mit der erfindungsgemässen Vorrichtung können auch Endoprothesen mit stärkerer Expansionskraft komprimiert und in das Lumen des Aussenkatheters eingeführt werden. Weiterhin hat sich überraschend gezeigt, dass die mit der erfindungsgemässen Vorrichtung komprimierten Endoprothesen nach dem Freisetzen ohne bleibende Verformungen des rohrförmigen Drahtgewebes selbsttätig in die ursprüngliche Formgebung zurückgeführt werden und somit ein optimales Implantieren im Gefäss (Blutgefäss) gewährleistet ist.

Gemäss der Weiterbildung besteht die Möglichkeit, dass der Katheter als eine Baueinheit in die erfindungsgernässe Vorrichtung eingesetzt und in komplett montiertem Zustand mit der Endoprothese beschickt werden kann.

Weitere vorteilhafte Merkmale ergeben sich aus den abhängigen Patentansprüchen, der nachstehenden Beschreibung und der Zeichnung.

Ausführungsbeispiele der Erfindung werden nachstehend anhand der Zeichnung beschrieben. Es zeigt:
- **Fig.1**: ein in Seitenansicht dargestelltes Teilstück einer Endoprothese in radial expandiertem Zustand;
- **Fig.2**: die in Seitenansicht dargestellte Endoprothese gemäss Fig.1 in axial auseinandergezogenem Zustand;
- **Fig.3**: ein im Schnitt dargestelltes erstes Ausführungsbeispiel einer Vorrichtung zum Einführen der Endoprothese gemäss Fig.1 in das distale Endstück eines Katheters;
- **Fig.4**: das im Schnitt dargestellte distale Endstück des Katheters mit der anhand der Vorrichtung gemäss Fig.3 eingeführten Endoprothese;
- **Fig.5**: eine im Schnitt dargestellte Variante eines Kopfstücks für die Vorrichtung gemäss Fig.3;
- **Fig.6**: ein im Schnitt dargestelltes zweites Ausführungsbeispiel der Vorrichtung zum Einführen der Endoprothese in das distale Endstück eines Katheters;
- **Fig.7**: ein entlang einer Trennebene in Seitenansicht dargestelltes sowie mit zwei gelenkig miteinander verbundenen Teilstücken versehenes Kopfstück für die Vorrichtung gemäss Fig.6;
- **Fig.8**: das gemäss Pfeilrichtung A in Ansicht dargestellte Kopfstück gemäss Fig.7 mit den beiden entlang einer Trennlinie relativ zueinander zum Einführen des Katheters aufgeschwenkt dargestellten Teilstücken;
- **Fig.9**: einen Schnitt durch die Vorrichtung gemäss Fig.6 entlang der Linie IX-IX;
- **Fig.10**: einen Schnitt durch die Vorrichtung gemäss Fig.6 entlang der Linie X-X;
- **Fig. 11**: einen in Ansicht sowie kleinerem Massstab dargestellten Aufnahmekörper für ein Gehäuse der Vorrichtung gemäss Fig.6; und
- **Fig.12**: eine Variante des entlang der Trennebene in Seiten-Ansicht dargestellten und mit zwei gelenkig verbundenen Teilstücken versehenen Kopfstücks für die Vorrichtung gemäss Fig.6.

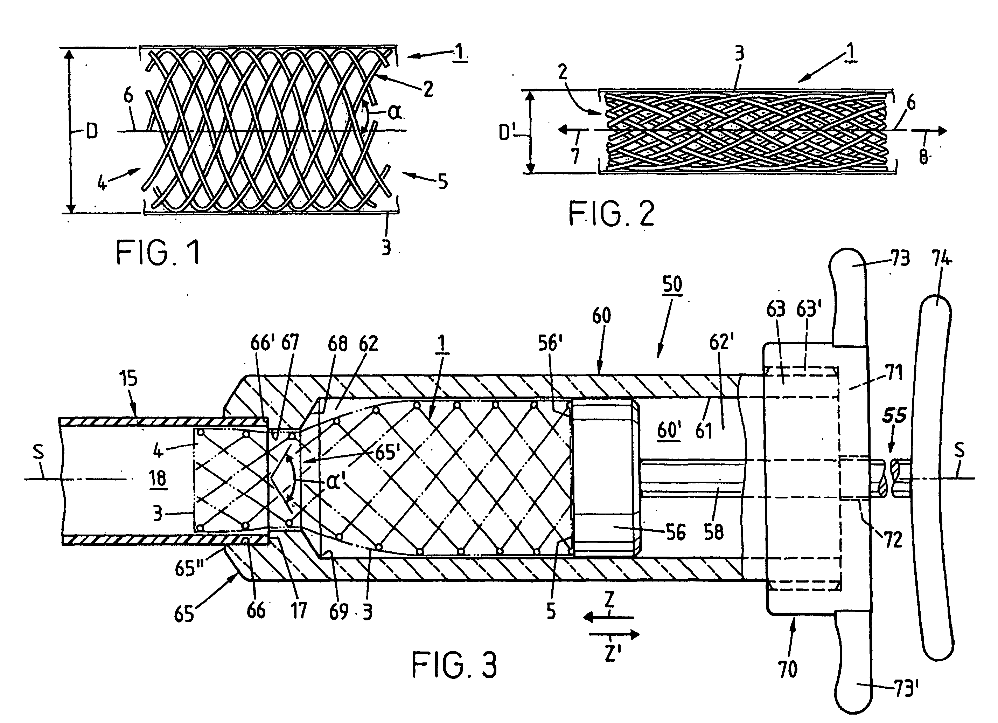

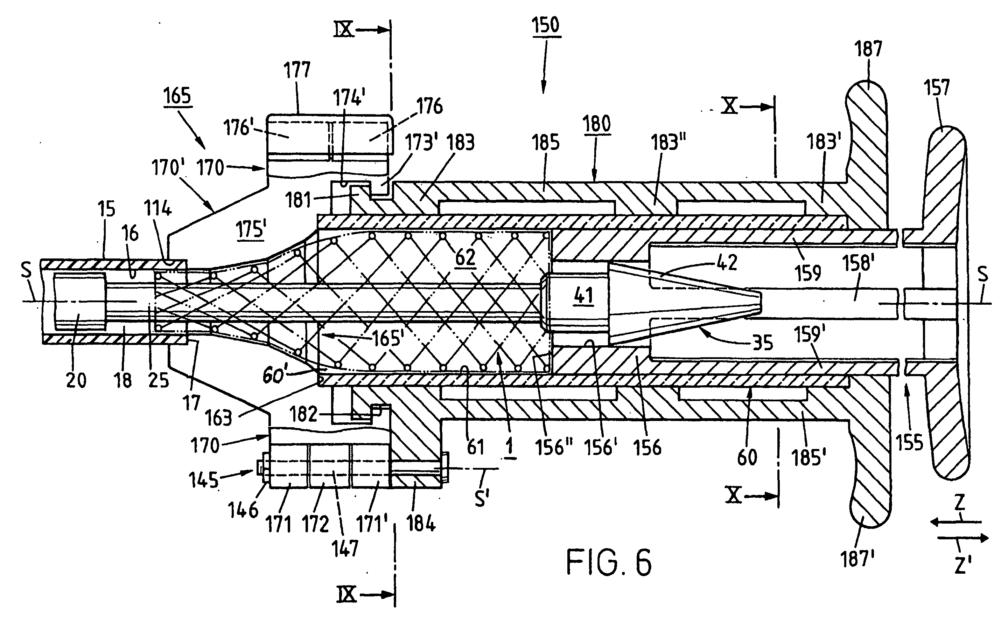

Fig.1 zeigt als Ausführungsbeispiel ein Teilstück einer rohrförmigen, selbstexpandierenden Endoprothese 1 in expandiertem und Fig.2 in radial komprimiertem und dadurch in der Länge gestreckten Zustand, wobei eine derartige Endoprothese beispielsweise aus der Druckschrift (US-A 4,655,771) bekannt ist. Zum Implantieren in das Gefäss eines Lebewesens wird die Endoprothese 1 mittels einer erfindungsgemässen Vorrichtung gemäss Fig.3 oder Fig.6 im Durchmesser verjüngt in das distale Endstück eines Katheters eingeführt. Die in an sich bekannter Weise selbstexpandierend ausgebildete Endoprothese 1 besteht im wesentlichen aus einem um eine theoretische Mittelachse 6 etwa schraubenlinig gewundenen Drahtgewebe 2 (Fig.1). Das Drahtgewebe 2 kann aussenseitig zusätzlich mit einer dehnbaren, schematisch dargestellten schlauchförmigen Hülle 3 versehen werden. In expandiertem Zustand (Fig.1) hat die Endoprothese 1 einen ersten, grösseren Durchmesser D, wobei die Endoprothese 1 zum Einführen in das distale Endstück des Katheters, wie in Fig.2 dargestellt, auf einen zweiten, kleineren Durchmesser D' verjüngt wird. Die Verjüngung der Endoprothese kann beispielsweise durch eine relativ geringe Zugwirkung an den beiden Enden 4 und 5 in Pfeilrichtung 7 und 8 erreicht werden. Nach Loslassen der beiden Enden 4 und 5 hat die Endoprothese 1 das Bestreben durch die eigene federelastische Rückstellkraft selbsttätig in die ursprüngliche Form (Fig.1) zurückkehren zu wollen. In expandiertem Zustand sind die Drähte beispielsweise unter stumpfem Winkel α zueinander angeordnet um eine möglichst hohe radiale Stützkraft zu erzeugen.

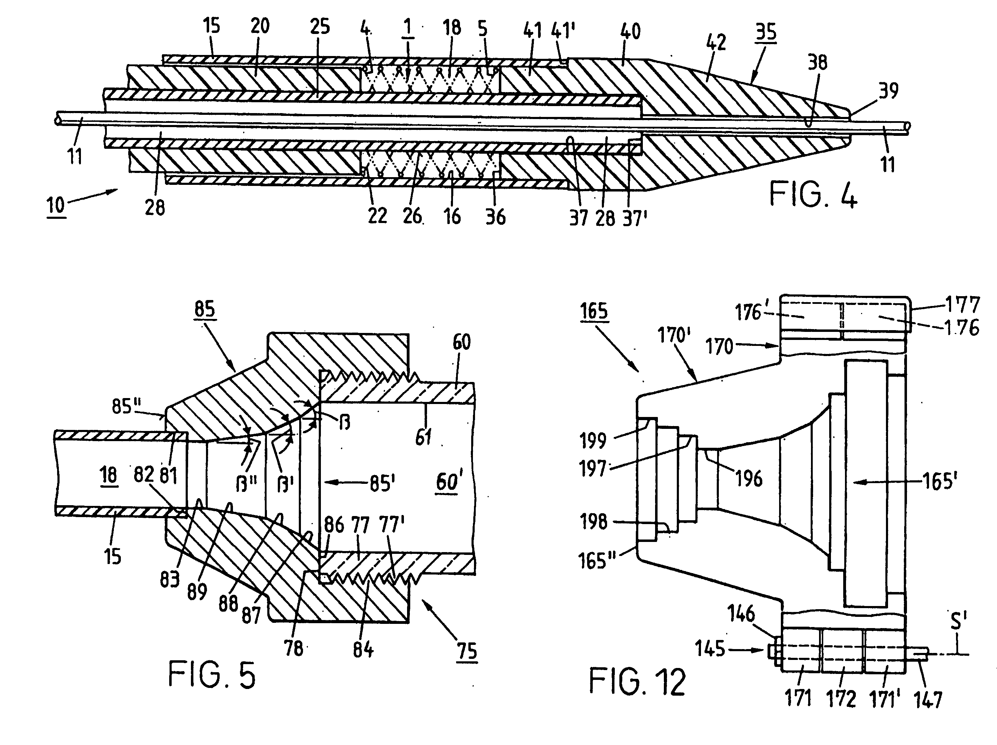

Fig.3 zeigt als erstes Ausführungsbeispiel eine im Schnitt dargestellte Vorrichtung 50. Die Vorrichtung 50 ist zum Einsetzen oder Einführen der hinsichtlich des Durchmessers D (Fig.1) vorbestimmten sowie hinsichtlich der Länge entsprechend abgeschnittenen Endoprothese 1 in ein Lumen 18 eines Aussenkatheters 15 ausgebildet. Der mit dem distalen Ende beziehungsweise mit der Stirnseite 17 in ein Kopfstück 65 der Vorrichtung 50 eingeschobene oder eingesetzte Aussenkatheter 15 ist ein Teilstück eines in Fig. 3 nicht näher dargestellten Katheters. Der Katheter wird nachstehend in Verbindung mit Fig.4 noch im einzelnen beschrieben.

Die im dargestellten Ausführungsbeispiel gemäss Fig.3 etwa in Form einer Injektionsspritze ausgebildete Vorrichtung 50 umfasst ein hohlzylindrisches Gehäuse 60, dessen Innenraum 60' durch eine darin angeordnete Kolben einheit 55 im wesentlichen in eine Vorderkammer 62 zur Aufnahme der Endoprothese 1 sowie in eine Hinterkammer 62' unterteilt ist. Die Kolben einheit 55 ist mittels einer daran angeordneten Kolbenstange 58 sowie eines am Ende daran befestigten und beispielsweise scheibenförmig ausgebildeten Griffelements 74 gemäss Pfeilrichtung Z oder Z' in axialer Richtung in dem Gehäuse 60 verschiebbar. Der an der Innenseite 61 des zylindrischen Innenraums 60' gegen Verkanten geführte Kolben 56 ist mit nicht dargestellten Mitteln, beispielsweise durch eine Schraubverbindung auswechselbar an der Kolbenstange 58 befestigt.

An dem hinteren Ende des Gehäuses 60 ist ein mit einem Aussengewinde 63' versehener Absatz 63 angeformt, auf welchen eine mit einem nicht näher dargestellten Innengewinde versehene Verschlusskappe 70 aufgeschraubt ist. In der Rückwand 71 der Verschlusskappe 70 ist zum Durchführen der Kolbenstange 58 eine Durchgangsöffnung 72 vorgesehen. Zur besseren Handhabung sind an der aufschraubbaren Verschlusskappe 70 radial nach aussen orientierte Griffelemente 73,73' angeordnet und in nicht dargestellter Weise befestigt. Die Griffelemente 73 und 73' sind dem scheibenförmigen Griffteil 74 entsprechend zugeordnet. Das Griffteil 74 mit dem daran angeordneten Kolben 56 ist in bezug auf die Verschlusskappe 70 und Griffelemente 73,73' in Pfeilrichtung Z oder Z' verschiebbar.

Das an dem vorderen Ende der Vorrichtung 50 angeordnete Kopfstück 65 ist, wie in Fig.3 dargestellt, mit einer der Vorderkammer 62 zugewandten Ausnehmung 65' und einer damit in Verbindung stehenden Durchtrittsöffnung 67 sowie mit einer Ausnehmung 66 versehen. Die Ausnehmung 66 ist ausgehend von der Stirnseite 65'' des Kopfstücks 65 in axialer Richtung durch eine ringförmige Anlagekante 66' begrenzt und zur Aufnahme des bis zu der Anlagekante 66' einschiebbaren Aussenkatheters 15 (Fig.3) ausgebildet.

Die der Vorderkammer 62 zugewandte Ausnehmung 65' des Kopfstücks 65 ist ausgehend von der zylindrischen Innenwand 61 des Gehäuses 60 in Richtung der Durchtrittsöffnung 67 als eine konisch verjüngende Gleitwand 68 ausgebildet. Die in Richtung der Durchtrittsöffnung 67 unter einem Winkel α' geneigte Ausnehmung 65' bildet im wesentlichen einen Trichter, durch welchen die von dem Kolben 56 zugeführte Endoprothese 1 zwangsläufig von dem grösseren, ersten Durchmesser D (Fig.1) auf den kleineren, zweiten Durchmesser D' (Fig.2) verjüngt in das Lumen 18 des Aussenkatheters 15 eingeführt wird. Der vorzugsweise stumpfe Wikel α' der im Richtung der trichterformigen Ausnehmung 65' orientierten Gleitwand 68 liegt etwa in der Grössenordnung zwischen 90° und 120°. Bei einer nicht dargestellten Variante besteht die Möglichkeit, dass die Gleitwand 68 ausgehend von der zylindrischen Innenwand 61 des Gehäuses 60 etwa bogenförmig in Richtung der Durchtrittsöffnung 67 verjüngend ausgebildet ist.

Bei dem in Fig.3 dargestellten ersten Ausführungsbeispiel der Vorrichtung 50 wird bei abgeschraubter Verschlusskappe 70 und herausgezogener Kolben einheit 55 die jeweilige, in das Lumen 18 des Aussenkatheters 15 einzusetzende Endoprothese 1 in den Innenraum 60' des Gehäuses 60 eingeschoben und anschliessend die Kolben einheit 55 eingeführt und danach die Verschlusskappe 70 wieder aufgeschraubt. Die mit dem hinteren einen Ende 5 an der Stirnwand 56' des Kolbens 56 anliegende Endoprothese 1 wird durch die in Pfeilrichtung Z orientierte Bewegung des Kolbens 56 mit dem gegenüberliegenden vorderen Ende 4 infolge der Konischen Gleitwand 68 im Durchmesser verjüngt und dabei, wie in Fig.3 schematisch dargestellt, radial Komprimiert durch die Durchtrittsöffnung 67 des Kopfstücks 65 in das Lumen 18 des Aussenkatheters 15 geschoben. Die vorzugsweise am äusseren Umfang der Endoprothese 1 vorgesehene Hülle 3 besteht aus einem Werkstoff mit optimalen Gleiteigenschaften und bewirkt somit ein gleichmässiges Verjüngen und Einführen der Endoprothese 1 in das Lumen 18 des Aussenkatheters 15. Die Endoprothese 1 wird dabei von dem Kolben 56 soweit in Pfeilrichtung Z bewegt, bis der Kolben 56 mit der Stirnseite 56' mit dem von der zylindrischen Innenwand 61 zur geneigten Gleitfläche 68 gebildeten Übergang 69 in Eingriff gelangt. Nach dem Entfernen des Aussenkatheters 15 von der Vorrichtung 50 wird das noch aus dem Lumen 18 herausragende, relativ kurze Teilstück der Endoprothese 1 manuell in den Aussenkatheter 15 geschoben und anschliessend der Katheter 10 (Fig.4) vollständig montiert.

Fig.4 zeigt in grösserem Massstab und zur allgemeinen Übersicht das im Schnitt dargestellte distale Endstück des an sich bekannten Katheters 10 mit der mittels der Vorrichtung 50 (Fig.3) in das Lumen 18 des Aussenkatheters 15 eingeführten Endoprothese 1. Bei dem dargestellten Ausführungsbeispiel umfasst der Katheter 10 den flexiblen Aussenkatheter 15, einen koaxial darin angeordneten und ebenfalls flexibel ausgebildeten Innenkatheter 20 sowie einen koaxial darin angeordneten Innenschaft 25. An dem distalen Ende des flexiblen Innenschafts 25 ist eine mit einer Ausnehmung 37 versehene Katheterspitze 35 angeordnet und beispielsweise durch eine geeignete Klebverbindung befestigt. Die Teile 35 und 25 bilden dabei eine in axialer Richtung retativ zu dem Innenkatheter 20 verschiebbare Baueinheit.

Zwischen der zylindrischen Innenwand 16 des Aussenkatheters 15 und der zylindrischen Aussenwand 26 des freien Teilstücks des Innenschafts 25 ist das in axialer Richtung orientierte Lumen 18 für die einsetzbare Endoprothese 1 vorgesehen. In montiertem Zustand liegt die Endoprothese 1 mit dem einen Endstück 4 an der Stirnseite 22 des Innenkatheters 20 und mit dem anderen Endstück 5 an der Rückseite 36 der Katheterspitze 35 an.

Die Katheterspitze 35 ist distalseitig mit einem bis zu einer Stirnkante 39 konisch verjüngend ausgebildeten Teilstück 42 versehen, an welches sich ein erstes zylindrisches Teilstück 40 sowie ein abgesetzt dazu ausgebildetes und mit einer Anlagekante 41' versehenes zweites zylindrisches Teilstück 41 anschliesst. Die Katheterspitze 35 ist ausgehend von der Stirnseite 39 mit einer in axialer Richtung orientierten Bohrung 38 versehen, welche in die Ausnehmung 37 der Katheterspitze 35 beziehungsweise in den Raum 28 des hohlzylindrischen Innenschafts 25 mündet. Der Katheter 10 kann weiterhin mit einem den Innenschaft 25 sowie die Katheterspitze 35 in axialer Richtung durchdringenden Führungsdraht 11 versehen sein. Das zylindrische Teilstück 41 der Katheterspitze 35 ist in axialer Richtung derart bemessen, dass in montiertem Zustand zwischen der Stirnseite 36 der Katheterspitze 35 und der Stirnkante 22 des Innenkatheters 20, wie in Fig.4 dargestellt, ein etwa der Länge der komprimierten Endoprothese 1 entsprechend ausgebildeter Abstand (nicht bezeichnet) vorgesehen ist.

Für die nicht näher dargestellte Implantation der Endoprothese 1 wird der Katheter 10 in an sich bekannter Weise in ein nicht dargestelltes Gefäss eines Lebewesens eingeführt. Sobald die entsprechende Stelle erreicht ist, wird der Aussenkatheter 15 relativ zu der Katheterspitze 35 zurückgezogen, wodurch die Endoprothese 1 aufgrund der eigenen federelastischen Rückstellkraft auf den vorgegebenen Aussendurchmesser ausgedehnt und freigegeben wird. Nach der Freigabe der Endoprothese 1 und dem Vorschieben des Aussenkatheters 15 auf das zylindrische Teilstück 41 der Katheterspitze 35 kann der Katheter 10 wieder aus dem Gefäss entfernt werden.

Fig.5 zeigt als Variante des ersten Ausführungsbeispiels (Fig.3) eine Vorrichtung 75 und man erkennt ein Teilstück des zylindrischen Gehäuses 60 ohne die darin verschiebbar angeordnete Kolben einheit 55. Das Gehäuse 60 ist abweichend von dem Ausführungsbeispiel gemäss Fig.3 an dem vorderen Ende mit einem Absatz 77 und einem Aussengewinde 77' versehen, auf welches ein mit einem Innengewinde 84 versehenes Kopfstück 85 aufschraubbar ist. Das mit einer inneren Anlagekante 86 versehene Kopfstück 85 wird bis zu der Stirnseite 78 auf das Gehäuse 60 geschraubt. An der Stirnseite 85'' des Kopfstücks 85 ist eine in bezug auf eine Durchtrittsöffnung 83 abgesetzt ausgebildete Ausnehmung 81 vorgesehen, welche in axialer Richtung durch eine ringförmige Anlagekante 82 begrenzt ist. Die Ausnehmung 81 dient zur Aufnahme des bis zu der Anlagekante 82 einschiebbaren Aussenkatheters 15. Der hohlzylindrische Innenraum 60' des Gehäuses 60 steht bei diesem Ausführungsbeispiel über eine mehrfach abgesetzte und im wesentlichen trichterförmig ausgebildete Ausnehmung 85' mit der Durchtrittsöffnung 83 und dem Lumen 18 des Aussenkatheters 15 in Verbindung.

Die in dem Kopfstück 85 vorgesehene und an der zylindrischen Innenwand 61 des Gehäuses 60 anschliessende Ausnehmung 85' ist zum Einführen des Endoprothese 1, wie beispielsweise in Fig.3 dargestellt mit einer ersten Gleitwand 87, einer daran anschliessenden zweiten Gleitwand 88 sowie einer daran anschliessenden dritten Gleitwand 89 versehen, wobei die dritte Gleitwand 89 in die zylindrische Durchtrittsöffnung 83 mündet. Bei der in axialer Richtung des Kopfstücks 85 orientierten, trichterförmigen Ausnehmung 85' ist die erste Gleitwand 87 in Richtung der zweiten Gleitwand 88 mit einem ersten Winkel β konisch verjüngend in Richtung der zweiten Gleitwand 88 und diese mit einem zweiten Winkel β' konisch verjüngend in Richtung der dritten Gleitwand 89 und diese mit einem dritten Winkel β" konisch verjüngend in Richtung der zylindrischen Durchtrittsöffnung 83 ausgebildet. Bei dem in Fig.5 dargestellten Ausführungsbeispiel der trichterförmigen Ausnehmung 85' ist der erste Winkel β grösser als der zweite Winkel β**'** und dieser grösser als der dritte Winkel β'' ausgebildet. Durch die besondere Ausgestaltung der Ausnehmung 85' in dem Kopfstück 85 wird eine sukzessive Verjüngung der zugeführten und in das Lumen 18 des Aussenkatheters 15 einzuführenden Endoprothese 1 (Fig.1) erreicht.

Bei der Vorrichtung 75 gemäss Fig.5 besteht in vorteilhafter Weise die Möglichkeit, in Abhängigkeit der jeweils einzuführenden Endoprothese ein Kopfstück 85 auf das Aussengewinde 77' des Gehäuses 60 aufzuschrauben. Das auswechselbar am Gehäuse 60 angeordnete Kopfstück 85 ist mit einer in Abhängigkeit der Endoprothese 1 entsprechend trichterförmig ausgebildeten Ausnehmung 85' versehene.

An dieser Stelle wird darauf hingewiesen, dass das eine zylindrische Gehäuse 60 mit angeformten dem Kopfstück 65 gemäss Fig.3 und das andere Zylindrische Gehäuse 60 mit dem aufschraubbaren Kopfstück 85 gemäss Fig.5 sowie ein nachstehend in Verbindung mit Fig.6 beschriebenes, zylindrisches Gehäuse 60 mit einem zweiteilig ausgebildeten Kopfstück 165 gemäss Fig.6 bis 8 und Fig.12 vorzugsweise jeweils aus transparentem Material hergestellt sind.

Fig.6 zeigt als zweites Ausführungsbeispiel eine schematisch und im Schnitt dargestellte Vorrichtung 150 zum Einsetzen oder Einführen der hinsichtlich der Länge vorbestimmt und entsprechend abgeschnittenen Endoprothese 1 in das Lumen 18 des Aussenkatheters 15. Die Endoprothese 1 ist beispielsweise analog dem Ausführungsbeispiel gemäss Fig.1 und Fig.2 ausgebildet, wobei aber andere Ausführungsvarianten derselben ebenfalls verwendbar sind.

Die Vorrichtung 150 umfasst einen rahmenförmig ausgebildeten Aufnahmekörper 180, das darin angeordnete und gelagerte Gehäuse 60, eine in dem Gehäuse 60 in Richtung der Längsachse S-S hin- und herbewegbare Kolbeneinheit 155 sowie das in der Gesamtheit mit 165 bezeichnete Kopfstück. Das Kopfstück 165 hat zwei, im dargestellten Ausführungsbeispiel vertikal in der Symmetrieebene voneinander getrennte Teilstücke 170 und 170', welche über eine im Abstand zu der Längsachse S-S angeordnete Gelenkstelle 145 miteinander verbunden an dem Aufnahmekörper 180 angeordnet sind. Die beiden Teilstücke 170 und 170' sind vorzugsweise über ein beispielsweise der Gelenkstelle 145 gegenüberliegend angeordnetes Verschlusselement 177 miteinander verriegelbar.

Der Aufnahmekörper 180 hat zwei in axialer Richtung orientierte und in Umfangsrichtung gegenüberliegend zueinander angeordnete Längsstege 185 und 185', welche am vorderen Ende durch ein erstes, zirkuläres Lagerteil 183 und am hinteren Ende durch ein zweites, zirkuläres Lagerteil 183' miteinander verbunden sind. Vorzugsweise ist in axialer Richtung etwa in der Mitte des Aufnahmekörpers 180 zusätzlich ein segmentförmig ausgebildetes Lagerteil 183" für das Gehäuse 60 vorgesehen. An dem zweiten Lagerteil 183' sind mindestens zwei gegenüberliegend zueinander angeordnete Griffteile 187 und 187' angeordnet, beispielsweise angeformt. Die einzelnen in axialer Richtung im Abstand zueinander angeordnete Lagerteile 183,183' und 183" des Aufnahmekörpers 180 dienen im wesentlichen zur Aufnahme und koaxialen Lagerung des Gehäuses 60. Das Gehäuse 60 ist mindestens teilweise, vorzugsweise aber vollständig aus transparentem Material hergestellt.

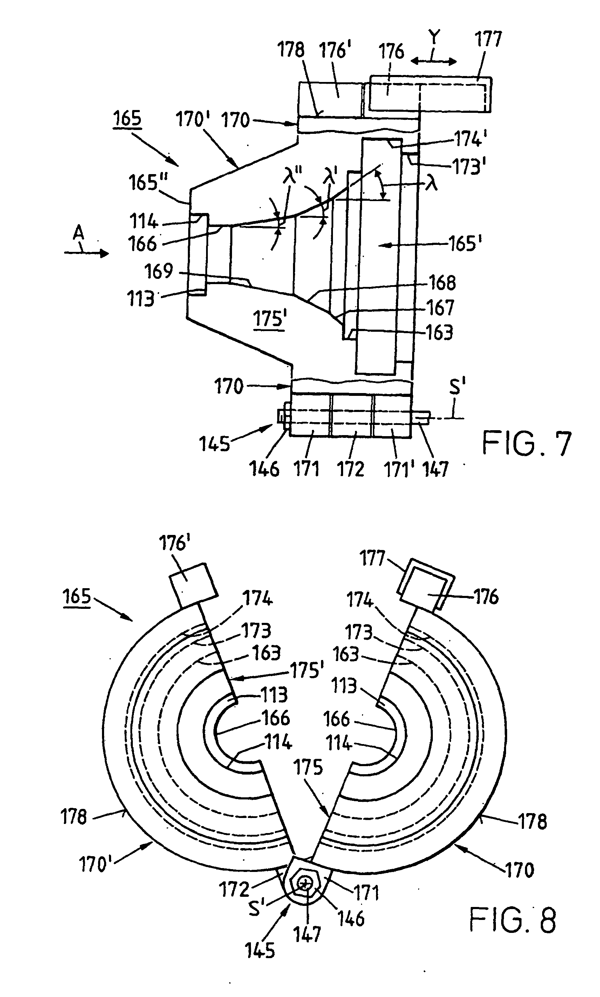

Das an dem anderen Ende der Vorrichtung 150 angeordnete und dem distalen Ende des Katheters 10 zugewandte Kopfstück 165 umfasst die beiden relativ zueinander aufschwenkbaren Teilstücke 170 und 170', welche um eine gemeinsame, parallel zur Längsachse S-S angeordneten Achse S' an einer Lasche 184 des Aufnahmekörpers 18 schwenkbar gelagert sind. Die beiden etwa schalenförmig ausgebildeten Teilstücke 170 und 170' sind mittels daran angeformter Lagerteile 171 und 171' beziehungsweise 172 über einen Gewindebolzen 147 und aufgeschraubter Mutter 146 an der Lasche 184 des Aufnahmekörpers 180 relativ zueinander schwenkbar gelagert (Fig.8). Die einzelnen Elemente 146,147, 171,171',172 bilden zusammen die in der Gesamtheit mit 145 bezeichnete Gelenkstelle.

An der dem Aufnahmekörper 180 zugewandten Seite sind die beiden schalenförmigen Teilstücke 170 und 170' je mit einem angeformten und jeweils halbkreisförmig ausgebildeten Ringelement 173 und 173' versehen. In zusammengeschwenktem Zustand der beiden Teilstücke 170 und 170' sind die beiden Ringelemente 173 und 173', wie in Fig.6 dargestellt, in einer Ringnut 182 des Aufnahmekörpers 180, sowie ein Ring 181 des Aufnahmekörpers 180 in einer Ringnut 174,174' der Teilstücke 170 und 170' angeordnet. In dieser Stellung ist das hohlzylindrische Gehäuse 60 in einer Ausnehmung 163 der beiden schalenförmigen Teilstücke 170 und 170' des Kopfstücks 165 angeordnet. Die beiden Teilstücke 170 und 170' sind jeweils mit einem am äusseren Durchmesser angeordneten Nocken 176 und 176' versehen und können durch das damit in Eingriff bringbare Schliesselement 177 miteinander verriegelt werden.

Abweichend von dem ersten Ausführungsbeispiel gemäss Fig.3 kann bei dem zweiten Ausführungsbeispiel gemäss Fig.6 das an dem rahmenförmig ausgebildeten Aufnahmekörper 180 angelenkte und mit den beiden relativ zueinander um die Achse S' schwenkbaren Teilstücken 170 und 170' versehene Kopfstück 165, wie bereits erwähnt, quer zu der Längsachse S-S der Vorrichtung 150 aufgeschwenkt werden. In der aufgeschwenkten Stellung der beiden Teilstücke 170 und 170' gemäss Fig.8 kann die Endoprothese 1 beispielsweise in völlig expandiertem Zustand in das in Fig.6 nicht bezeichnete Lumen des Gehäuses 60 eingeführt werden. Anschliessend wird der die Elemente 15, 20, 25 sowie 35 umfassende Katheter 10 als eine Baueinheit in einer ersten Phase in radialer Richtung zwischen die beiden aufgeschwenkten Teilstücke 170 und 170' (Fig.8) des Kopfstücks 165 eingeführt und anschliessend in axialer Richtung in das Gehäuse 60 der Vorrichtung 150 eingeschoben. Nachdem die Endoprothese 1 und der Aussenkatheter 15 in dem Gehäuse 60 angeordnet sind, werden die beiden Teilstücke 170 und 170' des Kopfstücks 165 wieder zusammengeschwenkt und mittels des Schliesselements 177 miteinander verriegelt (Fig.6)

Bei diesem Ausführungsbeispiel Kann der Katheter 10 in montierter Stellung mit dem Aussenkatheter 15 in eine ringförmige Ausnehmung 114 der beiden Teilstücke 170, 170' des Kopfstücks 165 gelagert werden. Die an dem Innenschaft 25 angeordnete Katheterspitze 35 steht vorzugsweise mit einem Kolben 156 der in Richtung der Längsachse S-S gemäss Pfeilrichtung Z oder Z' bewegbaren und in dem Gehäuse 60 der Vorrichtung 150 geführten Kolbeneinheit 155 in Wirkverbindung.

Die Kolbeneinheit 155 umfasst den mit einer Durchgangsöffnung 156'versehenen Kolben 156 sowie daran angeformte und in Richtung der Längsachse S-S orientierte Stege 158,158' und 159,159'. Die Kolbeneinheit 155 ist mittels der in Umfangsrichtung verteilt zueinander angeordneten Stege 158, 158' und 159,159' an der Innenwand 61 des Gehäuses 60 in Richtung der Längsachse S-S geführt. Am äusseren, dem Kolben 156 gegenüberliegenden Ende sind die Stege 158, 158' und 159,159' an einem im wesentlichen scheibenförmig ausgebildeten Griffelement 157 in nicht näher dargestellter Weise befestigt. Abweichend von dem Ausführungsbeispiel gemäss Fig.3 kann bei dem Ausführungsbeispiel gemäss Fig.6 der Katheter 10 (Fig.4) bei aufgeschwenkten Teilstücken 170,170' in montiertem zustand in das Kopfstück 165 und in das Gehäuse 60 eingeführt werden.

Zum Komprimieren und Einführen der Endoprothese 1 in das Lumen 18 des Aussenkatheters 15 wird die Kolbeneinheit 155 mit dem Griffelement 157 in Pfeilrichtung Z verschoben. Durch die in Pfeilrichtung Z orientierte Bewegung der Kolbeneinheit 155 wird die Endoprothese 1 durch eine etwa trichterförmig verjüngend ausgebildete Ausnehmung 165' des Kopfstücks 165, wie in Fig.6 teilweise dargestellt,in das Lumen 18 des Aussenkatheters 15 geschoben. Sobald die Kolbeneinheit 155 mit der Stirnseite 156" des Kolbens 156 mit der Ausnehmung 165' des Kopfstücks 165 in Eingriff gelangt, wird die Kolben einheit 155 in Pfeilrichtung Z' zurückgezogen und anschliessend das Kopfstück 165 durch Aufschwenken der beiden Teilstücke 170 und 170' für die Entnahme des Aussenkatheters 15 geöffnet. Anschliessend wird die Katheterspitze 35 mit dem Innenschaft 25 und dem angeformten zylindrischen Teilstück 41 manuell in das Lumen 18 des Aussenkatheters 15 geschoben.

Bei einer nicht dargestellten Variante besteht jedoch auch die Möglichkeit, dass bei der in Pfeilrichtung Z orientierten Schubbewegung der Kolbeneinheit 155 beispielsweise die Katheterspitze 35 mit dem Innenschaft 25 simultan in Pfeilrichtung Z bewegt wird. Hierbei kann die Katheterspitze 35 mit dem zylindrischen Teilstück 41 direkt in das Lumen 18 des Aussenkatheters 15 geschoben werden. Zur Erreichung der simultanen Bewegung der Kolbeneinheit 155 mit der Katheterspitze 35 und dem Innenschaft 25 ist die Durchgangsöffnung 156' in dem Kolben 156 beispielsweise mit einem nicht dargestellten Anschlag versehen, welcher mit dem konischen Teilstück 42 der Katheterspitze 35 in Eingriff steht und dadurch simultan mit der Kolbeneinheit 155 in Pfeilrichtung Z (Fig. 6) Schiebbar ist.

In Fig. 7 ist das Kopfstück 165 für die Vorrichtung 150 gemäss Fig.6 in der Trennebene dargestellt, und man erkennt die Fläche 175' des einen Teilstücks 170', das im oberen Bereich angeordnete und in Ansicht dargestellte Schliesselement 177 mit den zugeordneten Nocken 176 und 176' sowie die im unteren Bereich angeordnete und in Ansicht dargestellte Gelenkstelle 145 mit den einzelnen Teilen 146,147 und 171,171' sowie 172.

In Fig.8 ist das Kopfstück 165 gemäss der in Fig.7 eingezeichneten Pfeilrichtung A in Seitenansicht sowie entlang der theoretischen Trennebene in aufgeschwenktem Zustand dargestellt. Hierbei sind die beiden Teilstücke 170 und 170' zum Einsetzen des Katheters 10 (Fig.4) um die Achse S' der Gelenkstelle 145 teilweise aufgeschwenkt dargestellt. Weiterhin erkennt man in Fig.8 die an der kreisförmigen Oberfläche 178 der beiden Teilstücke 170 und 170' angeordneten Nokken 176 und 176' sowie das im Profilquerschnitt etwa U-förmig ausgebildete Schliesselement 177. Das Schliesselement 177 ist beispielsweise an dem einen Nocken 176 des ersten Teilstücks 170 in Doppelpfeilrichtung Y (Fig.7) verschiebbar angeordnet und kann mit dem anderen Nocken 176' des zweiten Teilstücks 170' derart in Eingriff gebracht werden, dass das Kopfstück 165 mit den aneinanderliegenden Flächen 175,175' der beiden Teilstücke 170 und 170' eine Einheit bildet.

Das Kopfstück 165 mit den beiden Teilstücken 170,170' ist, wie in Fig.7 und Fig.8 dargestellt, weiterhin zum Einführen der Endoprothese 1 in das Lumen 18 des Aussenkatheters 15 (Fig.6) mit der trichterförmig ausgebildeten Ausnehmung 165' versehen. Die Ausnehmung 165' steht über eine Durchtrittsöffnung 166 mit dem Lumen 18 des in Fig.7 und Fig.8 nicht dargestellten Aussenkatheters 15 in Verbindung. Die trichterförmige Ausnehmung 165' umfasst, wie beispielsweise in Fig.7 dargestellt, eine erste Gleitwand 167, eine daran anschliessende zweite Gleitwand 168 sowie eine dritte Gleitwand 169, wobei die dritte Gleitwand 169 in die Durchtrittsöffnung 166 mündet. Die an den beiden Teilstücken 170, 170' vorgesehenen Gleitwande 167, 168, 169 sowie die Öffnung 166 sind analog ausgebildet.

Bei der in axialer Richtung des Kopfstücks 165 orientierten, trichterförmigen Ausnehmung 165' ist die erste Gleitwand 167 in Richtung der zweiten Gleitwand 168 mit einem ersten Winkel λ konisch verjüngend in Richtung der zweiten Gleitwand 168 und diese Gleitwand mit einem zweiten Winkel λ' konisch verjüngend in Richtung der dritten Gleitwand 169 und diese mit einem dritten Winkel λ" konisch verjüngend in Richtung der zylindrischen Durchtrittsöffnung 166 ausgebildet. Bei dem dargestellten Ausführungsbeispiel (Fig.7) ist der erste Winkel λ vorzugsweise grösser als der zweite Winkel λ' und dieser vorzugsweise grösser als der dritte Winkel λ" ausgebildet. Weiterhin ist an der Stirnseite 165" des Kopfstücks 165 eine durch eine ringförmige Anlagekante 113 begrenzte Ausnehmung 114. zum Einführen bzw. Anschliessen des Aussenkatheters 15 (Fig.6) vorgesehen.

Die beiden Teilstücke 170,170' des Kopfstücks 165 sind weiterhin mit einer ersten ringförmigen Ausnehmung 163 für das Gehäuse 60 und mit der zweiten ringförmigen Ausnehmung 174 zur Aufnahme des am Aufnahmekörper 180 angeformten Anschlagrings 181 sowie mit dem Ringteil 173 versehen, welches in montiertem Zustand in die Ringnut 182 des Aufnahmekörpers 180 eingreift (Fig.6).

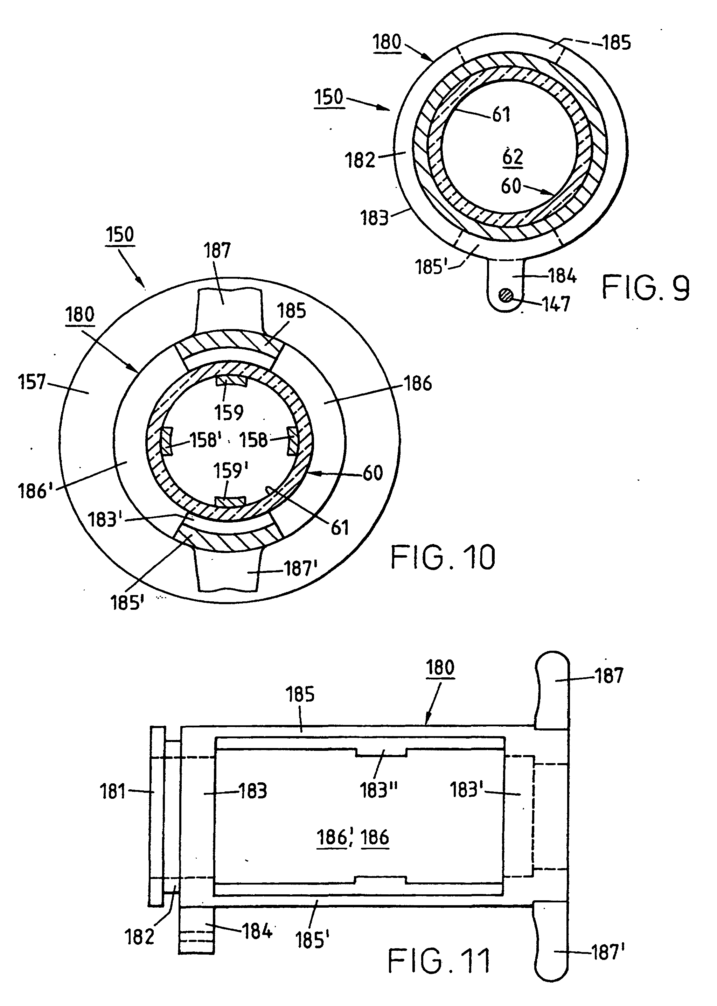

In Fig.9 ist die Vorrichtung 150 gemäss der in Fig.6 eingezeichneten Linie IX-IX im Schnitt dargestellt und man erkennt das Gehäuse 60, welches koaxial in den Lagerteilen 183,183' und 183'' des Aufnahmekörpers 180 gelagert ist. An dem Lagerteil 183 ist die Lasche 184 angeformt, an welcher die beiden Teilstücke 170,170' des Kopfstücks 165 mittels der Gelenkstelle 145 quer zur Längsachse S-S der Vorrichtung 150 aufschwenkbar gelagert sind (Fig.8).

In Fig.10 ist die Vorrichtung 150 gemäss der in Fig.6 eingezeichneten Linie X-X im Schnitt dargestellt und man erkennt das zwischen den beiden diametral zueinander angeordneten und etwa bogensegmentförmig ausgebildeten Längsstegen 185 und 185' des Aufnahmekörpers 180 gelagerte Gehäuse 60.

Weiterhin erkennt man in Fig.10 die an der Innenwand 61 des Gehäuses 60 anliegenden und in axialer Richtung orientierten sowie diametral zueinander angeordneten Stege 158,158' und 159,159' der Kolbeneinheit 155 (Fig.6). An den beiden Längestegen 185 und 185' des Aufnahmekörpers 180 sind die beiden gegenüberliegend zueinander angeordneten und hier nur teilweise dargestellten Griffteile 187 und 187' angeordnet. Weiterhin erkennt man das beispielsweise kreisscheibenförmig ausgebildete Griffteil 157 der Kolbeneinheit 155.

Fig.11 zeigt den in Ansicht dargestellten Aufnahmekörper 180 der Vorrichtung 150 und man erkennt die beiden an dem einen Ende des zweiten Lagerteils 183' angeordneten Griffteile 187 und 187' sowie die beiden Längsstege 185 und 185', welche an dem anderen Ende durch das zweite Lagerteil 183 miteinander verbunden sind. Das zweite Lagerteil 183 ist mit der äusseren Ringnut 182 und dem Anschlagring 181 sowie der angeformten Lasche 184 versehen. Die beiden zwischen den Lagerteilen 183,183' und 183'' angeordneten Längsstege 185 und 185' des Aufnahmekörpers 180 sind derart ausgebildet, dass in Umfangsrichtung wie in Fig.10 dargestellt, zwei gegenüberliegende Fenster in Form von in axialer Richtung orientierter Aussparungen 186 und 186' vorgesehen sind.

In Fig.12 ist als Variante das mit der Ausnehmung 165' versehene Kopfstück 165 für die Vorrichtung 150 gemäss Fig.6 dargestellt, welches die beiden Teilstücke 170 und 170', die daran am äusseren Umfang angeordneten 176, 176' mit dem zugeordneten Schliesselement 177 und die einzelnen gegenüberliegend angeordneten Elemente 146 ,147, 171, 171' und 172 der Gelenkstelle 145 umfasst. Die in dem Kopfstück 165 vorgesehene Ausnehmung 165' (Fig.7) ist analog der Ausnehmung 165' des Kopfstücks 165 gemäss Fig.12 ausgebildet.

Abweichend von dem Kopfstück 165 gemäss Fig.7 ist das Kopfstück 165 gemäss Fig.12 an der Stirnseite 165" mit mehreren im Durchmesser unterschiedlich dimensionierten Ausnehmungen für unterschiedlich ausgebildete Aussenkatheter. In Fig.12 sind die in dem einen Teilstück 170' angeordneten Ausnehmungen 197, 198 und 199 dargestellt, welche mit den in dem anderen Teilstück 170 des Kopfstücks 165 angeordneten Ausnehmungen (nicht dargestellt) korrespondierend übereinstimmen. Die Ausnehmungen sind jeweils zum Einführen des im Aussendurchmesser entsprechend dimensionierten Aussenkatheters 15 (Fig.6) ausgebildet.

Die Erfindung ist nicht auf die vorstehend beschriebenen Ausführungsbeispiele der Vorrichtung 50 mit dem Kopfstück 65 oder 85 beziehungsweise auf die Vorrichtung 150 mit dem Kopfstück 165 beziehungsweise auf die Ausführungsform des Kopfstücks 165 gemäss Fig.12 beschrankt. Weitere zweckmässige Ausgestaltungen der Vorrichtungen 50 und 150, welche zum Einführen eines länglichen Katheters 10, insbesondere eines Katheters 10 in montiertem Zustand ausgebildet sind, können ebenfalls Anwendung finden. Die Vorrichtung kann dabei auch derart ausgebildet werden, dass zwei in Längsrichtung getrennte halbschalenförmige Teilstücke am proximalen Ende der Vorrichtung zum Einsetzen des Katheters 10 aufschwenkbar miteinander wirkverbunden und am distalen Ende durch geeignete Mittel miteinander verriegelbar sind. Mit einer derartig ausgebildeten Vorrichtung kann der Katheter 10 mit relativ zur Katheterspitze 35 zurückgezogenem Aussenkatheter 15 als eine Baueinheit in die Vorrichtung eingesetzt und anschliessend die entsprechend ausgebildete Endoprothese 1 in den Aussenkatheter 15 eingeführt werden.

Mit der erfindungsgemässen Vorrichtung 50 beziehungsweise 150 können handelsübliche Katheter, welche zum Implantieren einer Endoprothese in ein Gefäss einzuführen sind, mit entsprechend ausgebildeten Endoprothesen beschickt (geladen) werden. Als besonders vorteilhaft wird die Vorrichtung 150 angesehen, da hierbei der Katheter in komplett montiertem Zustand mit der Endoprothese beschickt werden kann.

## Patentansprüche

1. Vorrichtung zum Einführen einer rohrförmigen, selbstexpandierenden Endoprothese in einen Katheter, mit einem Gehäuse (60) zur Aufnahme der Endoprothese (1) sowie einer Kolbeneinheit (55;155), mittels welcher die Endoprothese (1) in axialer Richtung in dem Gehäuse (60) verschiebbar und in das Lumen (18) eines hohlzylindrischen Aussenkatheters (15) des Katheters (10) einführbar ist, **dadurch gekennzeichnet, dass** dem Gehäuse (60) ein zum radialen Komprimieren der Endoprothese (1) ausgebildetes Kopfstück (65;85;165) zugeordnet ist, welches eine von dem hohlzylindrischen Innenraum (60') des Gehäuses (60) in Richtung einer in Bezug auf den Innenraum (60') kleiner ausgebildeten Durchtrittsöffnung (67;83;166) trichterförmige Ausnehmung (65';85';165') aufweist.

2. Vorrichtung nach Anspruch 1, **dadurch gekennzeichnet, dass** das mit der trichterförmigen Ausnehmung (65') versehene Kopfstück (65) an dem Gehäuse (60) angeformt ist und die Ausnehmung (65') sowie eine ausgehend von dem hohlzylindrischen Innenraum (60') unter stumpfen Winkel (α) in Richtung der Durchtrittsöffnung (67) orientierte kegelstumpfförmig ausgebildete Gleitwand (68) aufweist.

3. Vorrichtung nach Anspruch 2, **dadurch gekennzeichnet, dass** der stumpfe Winkel (α) der kegelstumpfförmigen Gleitwand (68) zwischen 90° und 120° beträgt.

4. Vorrichtung nach Anspruch 1, **dadurch gekennzeichnet, dass** das mit der trichterförmigen Ausnehmung (85') versehene Kopfstück (85) durch eine Schraubverbindung (84,77') auswechselbar an dem Gehäuse (60) angeordnet ist und die Ausnehmung (85') ausgehend von dem hohlzylindrischen Innenraum (60') mehrere jeweils unter spitzem Winkel (β,β'β") zueinander in Richtung der Durchtrittsöffnung (83) orientierte und kegelstumpfförmig ausgebildete Gleitwände (87,88,89) aufweist.

5. Vorrichtung nach Anspruch 4, **dadurch gekennzeichnet, dass** der erste spitze Winkel (β) der ersten Gleitwand (87) grösser als der zweite spitze Winkel (β') der zweiten Gleitwand (88) und der zweite spitze Winkel (β') grösser als der dritte spitze Winkel (β") der in Richtung der Durchtrittsöffnung orientierten Gleitwand (89) ausgebildet ist.

6. Vorrichtung nach Anspruch 1, **dadurch gekennzeichnet, dass** die mit einem Kolben (56) und einer Kolbenstange (58) sowie einem Griffteil (74) versehene Kolbeneinheit (55) durch eine an dem dem Kopfstück (65;85) gegenüberliegenden Ende des Gehäuses (60) angeordnete Verschlusskappe (70) gesichert und diese zum Einführen der Endoprothese (1) von dem Gehäuse (60) entfernbar ist.

7. Vorrichtung nach Anspruch 6, **dadurch gekennzeichnet, dass** die Verschlusskappe (70) auf das mit einem Absatz (63) und Aussengewinde (63') versehene Gehäuse (60) aufschraubbar ist.

8. Vorrichtung nach Anspruch 1, **gekennzeidhnet durch** einen Aufnahmekörper (180) zur Aufnahme des mit der Kolbeneinheit (155) versehenen Gehäuses (60) sowie des mit der Ausnehmung (165') und der Durchtrittsöffnung (166) versehenen Kopfstücks (165), welches entlang der Symmetrieebene in zwei schalenförmig ausgebildete und an einer Gelenkstelle (145) gelagerte Teilstücke (170,170') teilbar ist.

9. Vorrichtung nach Anspruch 8, **dadurch gekennzeichnet, dass** die beiden Teilstücke (170,170') des Kopfstücks (165) relativ zu dem Aufnahmekörper (180) und dem Gehäuse (60) zwischen einer Offenstellung und einer Schliessstellung an der Gelenkstelle (145) schwenkbar gelagert sind.

10. Vorrichtung nach einem der Ansprüche 8 und 9, **dadurch gekennzeichnet, dass** die beiden Teilstücke (170,170') am äusseren Umfang jeweils mit einem angeformten und der Gelenkstelle (145) gegenüberliegend angeordneten Nocken (176,176') sowie mit einem damit in Eingriff bringbaren Schliesselement (177) versehen sind, mittels welchem die beiden Teilstücke (170,170') des Kopfstücks (165) miteinander verriegelbar sind.

11. Vorrichtung nach Anspruch 8, **dadurch gekennzeichnet, dass** das mit der trichterförmigen Ausnehmung (165') und den beiden Teilstücken (170,170') versehene Kopfstück (165) ausgehend von dem hohlzylindrischen Innenraum (60') des Gehäuses (60) mit mehreren jeweils unter spitzem Winkel (λ,λ',λ") zueinander in Richtung der Durchtrittsöffnung (166) orientierten und kegelstumpfförmig ausgebildeten Gleitwänden (167,168,169) versehen sind.

12. Vorrichtung nach Anspruch 11, **dadurch gekennzeichnet, dass** der erste spitze Winkel (λ) der ersten Gleitwand (167) grösser als der zweite spitze Winkel (λ') der zweiten Gleitwand (168) und der zweite spitze Winkel (λ') grösser als der dritte spitze Winkel (λ") der in Richtung der Durchtrittsöffnung (166) orientierten Gleitwand (169) ausgebildet ist.

13. Vorrichtung nach Anspruch 8, **dadurch gekennzeichnet, dass** das mit den beiden Teilstücken (170,170') versehene Kopfstück (165) an der Stirnseite (165") eine zu der Durchtrittsöffnung (166) koaxial angeordnete und zur Aufnahme des mit dem zugewandten Endstück einführbaren Aussenkatheters (15) ausgebildete Ausnehmung (114) aufweist.

14. Vorrichtung nach Anspruch 13, **dadurch gekennzeichnet, dass** das Kopfstück (165) an der Stirnseite (165") mehrere zu der Durchtrittsöffnung (166) koaxial angeordnete und zur Aufnahme im äusseren Durchmesser unterschiedlich ausgebildeter Aussenkatheter (15) abgestufte Ausnehmungen (197,198;199) aufweist.

15. Vorrichtung nach Anspruch 8, **dadurch gekennzeichnet, dass** die Kolbeneinheit (155) einen mit einer axialen Ausnehmung (156') zum Einführen einer Katheterspitze (35) ausgebildeten Kolben (156) aufweist, welcher mittels am Umfang verteilt angeordneter und in axialer Richtung orientierter Stege (158,158';159,159') mit einem Griffelement (157) wirkverbunden ist.

16. Vorrichtung nach einem der Ansprüche 8 und 15, **dadurch gekennzeichnet, dass** die Endoprothese (1) entweder bei herausgezogener Kolbeneinheit (155) oder aber bei aufgeschwenkten Teilstücken (170,170') des Kopfstücks (165) in den hohlzylindrischen Innenraum (60') des Gehäuses (60) einführbar ist.

17. Vorrichtung nach Anspruch 1, **dadurch gekennzeichnet, dass** das Gehäuse (60) mindestens teilweise vorzugsweise aber vollständig aus transparentem Material hergestellt ist.

## Claims

1. Device for introducing a tubular, self-expanding endoprosthesis into a catheter, with a housing (60) for receiving the endoprosthesis (1) and with a piston unit (55;155) of which the endoprosthesis (1) can be displaced in the axial direction in the housing (60) and can be inserted into the lumen (18) of a hollow cylindrical outer catheter (15) of the catheter (10), **characterized in that** the housing (60) is assigned a head part (65;85;165) which is designed for radially compressing the endoprosthesis (1) and which has a funnel-shaped recess (65';85';165') extending from the hollow cylindrical interior (60') of the housing (60) in the direction of an outlet opening (67;83;166) which is smaller in relation to the interior (60').

2. Device according to claim 1, **characterized in that** the head part (65) provided with the funnel-shaped recess (65') is integrally formed on the housing (60) and has the recess (65') and also a slide wall (68) of frustoconical shape oriented from the hollow cylindrical interior (60') at an obtuse angle (α) in the direction of the outlet opening (67).

3. Device according to claim 2, **characterized in that** the obtuse angle (α) of the frustoconical slide wall (68) is between 90° and 120°.

4. Device according to claim 1, **characterized in that** the head part (85) provided with the funnel-shaped recess (85') is arranged on the housing (60) so as to be able to be replaced by means of a screw connection (84;77'), and the recess (85') has, starting from the hollow cylindrical interior (60'), a plurality of slide walls (87,88,89) of frustoconical shape each oriented at an acute angle (β,β',β'') to one another in the direction of the outlet opening (83).

5. Device according to claim 4, **characterized in that** the first acute angle (β) of the first slide wall (87) is greater than the second acute angle (β') of the second slide wall (88), and the second acute angle (β') is greater than the third acute angle (β") of the slide wall (89) oriented in the direction of the outlet opening (83).

6. Device according to claim 1, **characterized in that** the piston unit (55) provided with a piston (56) and with a piston rod (58) and with a grip part (74) is secured by a closure cap (70) arranged on that end of the housing (60) remote from the head part (65;85), and this can be removed from the housing (60) for introduction of the endoprosthesis (1).

7. Device according to claim 6, **characterized in that** the closure cap (70) can be screwed onto the housing (60) provided with a shoulder (63) and external thread (63').

8. Device according to claim 1, **characterized by** a receiving body (180) for receiving the housing (60) provided with the piston unit (155) and for receiving the head part (165) provided with the recess (165') and the outlet opening (166), which head part (165) can be divided along the plane of symmetry into two sections (170,170') designed as shells and mounted on a hinge (145).

9. Device according to claim 8, **characterized in that** the two sections (170,170') of the head part (165) are mounted so as to be pivotable about the hinge (145) relative to the receiving body (180) and the housing (60) between an open position and a closed position.

10. Device according to either of claims 8 and 9, **characterized in that** the two sections (170,170') are each provided on the outer circumference with an integrally formed cam (176,176') arranged lying opposite the hinge (145), and also with a closure element (177) which can be brought into engagement with said cam and by means of which the two sections (170,170') of the head part (165) can be locked together.

11. Device according to claim 8, **characterized in that** the head part (165) provided with the funnel-shaped recess (165') and with the two sections (170,170') is provided, starting from the hollow cylindrical interior (60') of the housing (60), with a plurality of slide walls (167,168,169) which are of frustoconical shape and are oriented each at an acute angle (λ,λ',λ") to one another in the direction of the outlet opening (166).

12. Device according to claim 11, **characterized in that** the first acute angle (λ) of the first slide wall (167) is greater than the second acute angle (λ' ) of the second slide wall (168), and the second acute angle (λ') is greater than the third acute angle (λ") of the slide wall (169) oriented in the direction of the outlet opening (166).

13. Device according to claim 8, **characterized in that** the head part (165) provided with the two sections (170,170') has, on its front face (165"), a recess (114) which is arranged coaxially with respect to the outlet opening (166) and which is designed to receive the outer catheter (15) that can be introduced with the endpiece facing in front.

14. Device according to claim 13, **characterized in that** the head part (165) has, on its front face (165"), a plurallity of stepped recesses (197,198,199) which are arranged coaxially with respect to the outlet opening (166) and are designed to receive outer catheters (15) of different external diameter.

15. Device according to claim 8, **characterized in that** the piston unit (155) has a piston (156) designed with an axial recess (156') for insertion of a catheter tip (35), which piston (156) is operatively connected to a grip element (157) by means of ridges (158,158';159,159') distributed about the circumference and oriented in the axial direction.

16. Device according to either of claims 8 and 15, **characterized in that** the endoprosthesis (1) can be introduced into the hollow cylindrical interior (60') of the housing (60), either with the piston unit (155) pulled out or with the sections (170,170') of the head part (165) pivoted upwards.

17. Device according to claim 1, **characterized in that** the housing (60) is made at least partially, but preferably completely, of transparent material.

## Revendications

1. Dispositif d'introduction d'une endoprothèse tubulaire à expansion automatique dans un cathéter, comportant un logement (60) destiné à recevoir l'endoprothèse (1) ainsi qu'une unité à piston (55;155) au moyen de laquelle l'endoprothèse (1) peut être poussée dans le logement (60) dans une direction axiale et peut être introduite dans le lumen (18) d'un cathéter externe cylindrique creux (15) du cathéter (10), **caractérisé en ce qu'**est coordonné au logement (60) un élément de tête (65;85;165) conçu pour une compression radiale de l'endoprothèse (1), lequel élément présente un évidement en forme d'entonnoir (65';85';165') formé par l'espace interne cylindrique creux (60') du logement (60) dans la direction d'une ouverture de passage (67;83;166) conçue pour être plus petite que l'espace interne (60').

2. Dispositif selon la revendication 1, **caractérisé en ce que** l'élément de tête (65) muni de l'évidement en forme d'entonnoir (65') est façonné au niveau du logement (60) et présente l'évidement (65') et une paroi de glissement (68) en forme de cône tronqué, orientée, en partant de l'espace interne cylindrique creux (60'), selon un angle obtus (α) dans la direction de l'ouverture de passage (67).

3. Dispositif selon la revendication 2, **caractérisé en ce que** l'angle obtus (α) de la paroi de glissement en forme de cône tronqué (68) est compris entre 90° et 120°.

4. Dispositif selon la revendication 1, **caractérisé en ce que** l'élément de tête (85) muni de l'évidement en forme d'entonnoir (85') est disposé au niveau du logement (60) de façon amovible par un assemblage à vis (84,77'), et l'évidement (85'), en partant de l'espace interne cylindrique creux (60') présente plusieurs parois de glissement (87,88,89) formant un angle aigu (β,β',β") entre elles, orientées dans la direction de l'ouverture de passage (83) et conçues en forme de cône tronqué.

5. Dispositif selon la revendication 4, **caractérisé en ce que** le premier angle aigu (β) de la première paroi de glissement (87) est plus grand que le second angle aigu (β') de la seconde paroi de glissement (88), et que le second angle aigu (β') est plus grand que le troisième angle aigu β") de la paroi de glissement (89) orientée dans la direction de l'ouverture de passage.

6. Dispositif selon la revendication 1, **caractérisé en ce que** l'unité à piston (55) équipée d'un piston (56) et d'une tige de piston (58), ainsi que d'un élément de prise (74), est fixée par un capot de fermeture (70) disposé au niveau de l'extrémité opposée à l'élément de tête (65;85) du logement (60), et **en ce que** celui-ci peut être retiré du logement (60) pour l'introduction de l'endoprothèse (1).

7. Dispositif selon la revendication 6, **caractérisé en ce que** le capot de fermeture (70) peut être vissé sur le logement (60) muni d'un épaulement (63) et d'un filetage externe (63').

8. Dispositif selon la revendication 1, **caractérisé par** un corps de réception (180) destiné à la réception du logement (60) équipé de l'unité à piston (155), et de l'élément de tête (165) équipé de l'évidement (165') et de l'ouverture de passage (166), lequel élément de tête peut être scindé le long du plan de symétrie en deux éléments partiels (170,170') exécutés en forme de coque et fixés au niveau d'un point d'articulation (145).

9. Dispositif selon la revendication 8, **caractérisé en ce que** les deux éléments partiels (170,170') de l'élément de tête (165) sont fixés de façon à pivoter au niveau du point d'articulation (145) par rapport au corps de réception (180) et au logement (60), entre une position d'ouverture et une position de fermeture.

10. Dispositif selon l'une des revendications 8 et 9, **caractérisé en ce que** les deux éléments partiels (170,170') sont munis, au niveau de la périphérie externe, respectivement d'une came (176,176') façonnée et disposée à l'opposé du point d'articulation (145), et d'un élément de fermeture (177) pouvant être amené en prise et au moyen duquel les deux éléments partiels (170,170') de l'élément de tête (165) peuvent être verrouillés ensemble.

11. Dispositif selon la revendication 8, **caractérisé en ce que** l'élément de tête (165), équipé de l'évidement en entonnoir (165') et des deux éléments partiels (170,170'), en partant de l'espace interne cylindrique creux (60') du logement (60), est équipé de plusieurs parois de glissement (167,168,169) orientées les unes par rapport aux autres en formant un angle aigu (λ,λ',λ,") dans la direction de l'ouverture de passage (166), et exécutées en forme de cône tronqué.

12. Dispositif selon la revendication 11, **caractérisé en ce que** le premier angle aigu (λ) de la première paroi de glissement (167) est plus grand que le second angle aigu (λ') de la seconde paroi de glissement (168), et que le second angle aigu (λ') est plus grand que le troisième angle aigu (λ") de la paroi de glissement (169) orientée dans la direction de l'ouverture de passage.

13. Dispositif selon la revendication 8, **caractérisé en ce que** l'élément de tête (165) équipé des deux éléments partiels (170,170') présente au niveau de la face avant (165") un évidement (114) disposé coaxialement par rapport à l'ouverture de passage (166) et conçu pour recevoir le cathéter externe (15) pouvant être introduit avec l'élément d'extrémité.

14. Dispositif selon la revendication 13, **caractérisé en ce que** l'élément de tête (165) présente au niveau de la face avant (165") plusieurs évidements étagés (197;198;199) disposés coaxialement par rapport à l'ouverture de passage (166), et conçus pour loger des cathéters externes (15) de différents diamètres externes.

15. Dispositif selon la revendication 8, **caractérisé en ce que** l'unité à piston (155) présente un piston (156) exécuté avec un évidement axial (156') pour l'introduction d'une pointe de cathéter (35), lequel est en relation par action, au moyen de tiges (158,158';159,159') orientées dans la direction axiale et réparties sur la circonférence, avec un élément de prise (157).

16. Dispositif selon l'une des revendications 8 et 15, **caractérisé en ce que** l'endoprothèse (1) peut être introduite dans l'espace interne cylindrique creux (60') du logement (60) avec l'unité à piston (155) retirée ou avec les éléments partiels (170,170') de l'élément de tête (165) pivotés en ouverture.

17. Dispositif selon la revendication 1, **caractérisé en ce que** le logement (60) est fabriqué au moins partiellement, mais de préférence entièrement, avec un matériau transparent.
